Europäisches Patentamt

European Patent Office

Office européen des brevets

(19)

(11) Veröffentlichungsnummer: **0 148 380**
**B1**

(12) **EUROPÄISCHE PATENTSCHRIFT**

(45) Veröffentlichungstag der Patentschrift: **18.04.90**

(21) Anmeldenummer: **84114094.0**

(22) Anmeldetag: **22.11.84**

(51) Int. Cl.⁵: **B 01 J 19/08,** B 01 D 17/06,
B 01 D 43/00, A 61 L 2/02,
C 02 F 1/46

(54) **Elektroimpulsverfahren und Vorrichtung zur Behandlung von Stoffen.**

(30) Priorität: **09.12.83 DE 3344668**
**11.04.84 DE 3413583**

(43) Veröffentlichungstag der Anmeldung:
**17.07.85 Patentblatt 85/29**

(45) Bekanntmachung des Hinweises auf die
Patenterteilung:
**18.04.90 Patentblatt 90/16**

(84) Benannte Vertragsstaaten:
**AT BE CH DE FR GB IT LI LU NL SE**

(56) Entgegenhaltungen:
**EP-A-0 051 463**
**DE-A-1 642 793**
**DE-A-2 336 085**
**DE-A-2 907 887**
**DE-A-3 116 623**

Die Akte enthält technische Angaben, die nach
dem Eingang der Anmeldung eingereicht
wurden und die nicht in dieser Patentschrift
enthalten sind.

(73) Patentinhaber: **Doevenspeck, Heinz**
**Sigurdstrasse 1**
**D-4950 Minden (DE)**

(72) Erfinder: **Doevenspeck, Heinz**
**Sigurdstrasse 1**
**D-4950 Minden (DE)**

(74) Vertreter: **Bolte, Erich, Dipl.-Ing.**
**c/o Meissner & Bolte Patentanwälte Hollerallee**
**73**
**D-2800 Bremen 1 (DE)**

## Beschreibung

Die Erfindung betrifft ein Elektroimpulsverfahren zur Behandlung von dispersen Systemen, zur Gewinnung einzelner Phasen aus den dispersen Systemen oder zur Abtötung von Bakterien, bei denen die Stoffe in einem Elektrolyt mehrfach zeitlich aufeinanderfolgend Elektroimpulsen in Form von Kondensatorentladungen ausgesetzt werden, sowie ein Vorrichtung zur Durchführung des Verfahrens.

Aus der DE—A—16 42 793 ist es bekannt, daß man Lösungen aller Art mit Elektroimpulsen behandeln kann. Nach der Lehre dieser Druckschrift eignet sich das Verfahren nicht nur zum Entkalken von Wasser bzw. Entsalzen von Flüssigkeiten, sondern auch zur Stärkung der Widerstandsfähigkeit sämtlicher möglicher Organismen, zur Beschleunigung von Gär- und anderen biologischen Prozessen. Eine konkrete Lehre dahingehend, welche Art von Elektroimpulsen in welcher Weise angewandt werden soll, ist der Druckschift nicht zu entnehmen. Vielmehr lehrt die Druckschrift, daß sowohl Spannungsimpulse als auch Wechselspannungen und darüber hinaus auch Gleichspannungen einzeln und in Kombination verwendbar sind. Bezüglich der dort vorgeschlagenen Anwendung von Elektroimpulsen, wird in der Druckschrift vorgeschlagen, diese mit einer bestimmten Wiederholungsfrequenz anzuwenden, welche der behandelten Flüssigkeit in einer nicht näher beschriebenen Weise angepaßt sein soll.

Aus der DE—A—29 07 887 ist es bekannt, daß man Abwasser zur Entkeimung und/oder zur Entsalzung mit Elektroimpulsen konstanter Energie unter Zugabe von Chemikalien behandeln kann.

Aus der DE—A—23 36 085 ist es bekannt, daß man mittels einer Gleichspannung ein flüssiges Medium sterilisieren kann, wenn dieses eine Relativbewegung zu den dort vorgesehenen Elektroden ausführt.

Aus der DE—B—12 44 338 ist es bekannt, daß man organisches Material mittels Entladungen von hochgespannten Kondensatoren sterilisieren kann. Auch diese Druckschrift lehrt, man solle mit Elektroimpulsen lediglich einer einzigen Energiedichte arbeiten.

Verfahren und Vorrichtung der gattungsbildenden Art sind aus der DE—PS—12 37 541 bekannt. Diese Patentschrift beschreibt die Grundlagen des Elektroimpulsverfahrens, das zur vielfältigen Behandlung organischer und anorganischer Stoffe geeignet ist. Besondere Anwendungsgebiete des Elektroimpulsverfahrens liegen in der Gewinnung einzelner Phasen aus dispersen Systemen, was vor allem wesentliche Bedeutung für die Aufspaltung von Lebensmitteln hat, beispielsweise bei der Zuckergewinnung, der Trennung der einzelnen Phasen (Eiweiß, Fett, Wasser etc.) von Fleischprodukten usw. Weiterhin wurder dort bereits festgestellt (Sp8, Zeile 16—20), daß Bakterien (Mikroorganismen) hierdurch abgetötet werden können. Dies ist für die Haltbarmachung von Lebensmitteln und die Trinkwasser- sowie Abwasseraufbereitung von großer Bedeutung. Somit ist das Elektroimpulsverfahen eine echte Alternative zur Strahlungsbehandlung von Lebensmitteln, bei der mit sehr kurzwelliger Strahlung gearbeitet wird. Auch ist es bekannt, daß chemische Reaktionen durch Elektroimpulse ausgelöst werden können und daß auch Molekülketten organischer und anorganischer Substanzen gezüchtet werden können (Genmanipulation, Züchtung von Molekülketten beispielsweise zur Herstellung dünner Kohlefäden).

Das grundlegende Wesen des Elektroimpulsverfahrens liegt darin, die in einem Elektrolyt vorhandenen Stoffe bzw. die selbst einen Elektrolyt bildenden Stoffe kurzen elektrischen und/oder elektromagnetischen Feldern auszusetzen, um die gewünschten Reaktionen auszulösen. Diese Felder werden hierbei durch Entladung eines oder mehrerer Kondensatoren längs einer durch mindestens zwei Elektroden gebildeten Entladestrecke, in welcher die zu behandelnden Stoffe zusammen mit dem Elektrolyten als Dielektrikum wirken, erzeugt. Wichtig ist, daß die Elektroimpulse steile Anstiegsflanken haben. Je nach gewünschter Reaktion (Aufspalten disperser Systeme; Abtöten von Bakterien oder Zückten von Substanzen) wird mit unterschiedlichen Energien gearbeitet ("härte", "mittelweiche" und "weiche" Impulse).

Eine Schaltung zur Erzeugung solcher Elektroimpulse ist in der DE—PS 12 33 958 beschrieben. Über einen Netztranformator wird die Netzspannung hochtransformiert und über ein ventilartig wirkendes Thyratron aufgeladen. Zur Entladung der Kondensatoren können diese über Thyristoren mit den zugeordneten Entladestrecken verbunden werden. Wichtig ist, daß in der Aufladephase die Kondensatoren von den Entladestrekken abgetrennt sind, während sie in die Entladephase vom Netzteil abgetrennt sind.

Aus des DE—PS 19 46 267 ist es bekannt, in Bewegungsrichtung der Stoffe hintereinander angeordnete Entladestrecken (Elektroden) anzuordnen. Jede Entladestrecke (Elektroden mit Dielektrikum) wirkt hierbei als Kondensator.

Der DE—OS 29 07 887 ist zu entnehmen, daß einige chemische Reaktionen beim Elektroimpulsverfahren, insbesondere beim Abtöten von Bakterien (Abwasserreinigung) sowie Entsalzen von Lösungen, durch dosierte Zugabe von Oxidations- oder Reduktionsstoffen verbessert werden können. Dies hat auch seinen Niederschlag in dem Aufsatz von H. Hülsheger et al ("Radiat Environ Biophys", 1981, 20:53—56) gefunden sowie auch einem weiteren Aufsatz von H. Hülsheger et al ("Radiat Environ Biophys" 18, 281—288 (1980)). Dort wurde insbesondere die Tötungsrate von Bakterien beim Impulsverfahren in Abhängigkeit von Zusatzstoffen, Impulshöhe und Impulsdauer sowie Temperatur und Impulsfrequenz untersucht.

Die grundlegenden chemischen und molekularen Zusammenhänge des Elektroimpulsverfahrens sich auch in einem Aufsatz von Manfred Eigen "Die "unmeßbar" schnellen Reaktionen" (Reprint from Le Prix Nobel 1967, S. 151—180) beschrieben.

Ebenso befaßt sich J. Bernhardt in seinem Aufsatz "Biologische Wirkungen elektromagnetischer Felder" (Zeitung Naturforschung 34c, 616—627 (1979)) mit dem Elektroimpulsverfahren und beschreibt die Züchtung von Zellketten (Perlschnurketten) unter Einfluß eines elektrischen Feldes. Allerdings weist er darauf hin, daß gepulste Felder nicht wirksamer seien als kontinuierliche Felder mit derselben mittleren Feldenergie, was sich durch die vorliegende Erfindung nicht als richtig erweisen hat, da für einer einwandfreie Reaktion keine bleibenden Ionen (Radikale) erzeugt werden dürfen.

In der Zeitschrift UMSCHAU 78 (1978), Heft 2, S. 54 ist der Einfluß von Magnetfeldern auf chemische Reaktionen erwähnt.

In der Zeitschrift BIOELECTRO CHEMISTRY AND BIOENERGETICS 3, 58—83 (1976) ist der Einfluß externer elektrischer Felder auf Zellmembranen beschrieben und wiederum ein Hinweis auf kurze Elektroimpulse in einer Entladekammer eines Hochspannungskreises erwähnt. Hier wird auch davon gesprochen, daß der Widerstand der Membran für sehr kurze Stromimpulse proportional der Dicke der Membran ist.

Schließlich beschreibt die DE—OS 31 16 623 den Einfluß des fortlaufenden Umwälzens der Stoffe (durch einen "Wickelmodul") während der Elektroimpulsbehandlung.

Allen bisher angewandten Verfahren bzw. gebauten Vorrichtungen haftet jedoch der Nachteil an, daß die gewünschten Reaktionen nicht immer präzise genug ausgelöst werden, so daß die Ausbeute für eine großtechnische Ausnutzung nicht zufriedenstellend ist.

Aufgabe der Erfindung ist es daher, das gattungsbildende Verfahren sowie die gattungsbildende Vorrichtung dahingehend zu verbessern, daß eine höhere Ausbeute erzielt wird.

Diese Aufgabe wird verfahrensmäßig durch die im Kennzeichenteil des Anspruches 1 und vorrichtungsmäßig durch die im Kennzeichenteil des Patentranspruches 13 angegebenen Merkmale gelöst.

Der Grundgedanke der Erfindung liegt somit darin, während der Behandlung der Stoffe unterschiedliche Energiedichten anzuwenden, um selektiv und gezielt bestimmte Reaktionen auszulösen, insbesondere bestimmte Bakterienarten gezielt abzutöten. Es werden Impulsgruppen unterschiedlicher Energedichte angewandt, wobei eine Impulsgruppe eine beliebige Anzahl von Einzelimpulsen enthält, wobei auch der Fall umfaßt sein soll, bei dem eine "Impulsgruppe" nur einen einzigen Impuls enthält. Enthält eine Impulsgruppe mehrere Einzelimpulse, so haben diese Einzelimpulse jeweils die gleiche Energiedichte.

Der Erfindung liegt die Erkenntnis zugrunde, daß beispielsweise zum Abtöten verschiedener Bakterien unterschiedliche Energiedichten benötigt werden. Die Mindestenergiedichte hängt beispielsweise von der Größe der Zellstruktur, dem Alter der Zelle ab oderdan, ob diese GRAMnegativ oder GRAM-positiv ist. Beispielsweise lassen sich "junge" Zellen, die noch in der Wachstumsphase sind, mit geringeren Energiedichten abtöten als "ältere" Zellen. Weiterhin berücksichtigt die Erfindung, daß mehrere reaktionsparameter in der Praxis kaum exakt ermittelt werden können und sich während der Behandlung der Stoffe sogar noch ändern, was u. a. darauf zurückzuführen ist, daß durch vorhergehende Elektroimpulse beispielsweise die Dielektrizitätskonstante der Stoffe verändert wird. Durch Anwendung mehrerer Elektroimpulse unterschiedlicher Energiedichte werden damit diese Veränderungen ausgeglichen, da die Wahrscheinlichkeit erhöht wird, daß der die gewünschte Reaktion auslösende Elektroimpuls mit der "richtigen" Energiedichte ebenfalls vorhanden ist. Theoretisch wäre es zwar auch möglich, mit verhältnismäßig hohen Energiedichten, beispielsweise mit Feldstärken von 20—25 kV/cm zu arbeiten, womit mit Sicherheit die benötigte Energiedichte für praktisch alle Bakterien überschritten wäre; dies hat neben einem übermäßigen Energiever brauch den Nachteil, daß vereinzelte elektrische "Durchschläge" mit Plasmabildung und bleibender Ionisierung auftreten würden und daß sich der Elektrolyt stark erwärmen würde. Diese Erwärmung ist in vielen Anwendungsfällen unerwünscht, da sie das Behandlungsergebnis verfälscht und da aus wasserrechtlichen Gründen erwärmte Abwässer nicht in die Kanalisation oder in Flüsse abgeleitet werden dürfen.

Schließlich ist es bei der Abwasseraufbereitung auch gar nicht erwünscht, sämtliche Bakteiren abzutäten, da gewisse Arten von Bakterien bei einer späteren biologischen Abwasseraufbereitung wünschenswert sind und später wieder zugesetzt werden müßten.

Vorteilhafte Ausgestaltungen und Weiterbildungen der Erfindung sind den Unteransprüchen zu entnehmen.

Im folgenden wird die Erfindung anhand von Ausführungsbeispielen im Zusammenhang mit der Zeichnung ausführlicher erläutert. Es zeigt:

Fig. 1 eine Prinzipskizze einer Vorrichtung nach der Erfindung zur Behandlung von in Flüssigkeit gelösten Substanzen;

Fig. 2 eine Prinzipskisse einer Vorrichtung zur Behandlung pastöser Substanzen und

Fig. 3 ein Prinzipschaltbild einer elektrischen Schaltung, die bei den Vorrichtungen der Fig. 1 und 2 zur Anwendung kommt.

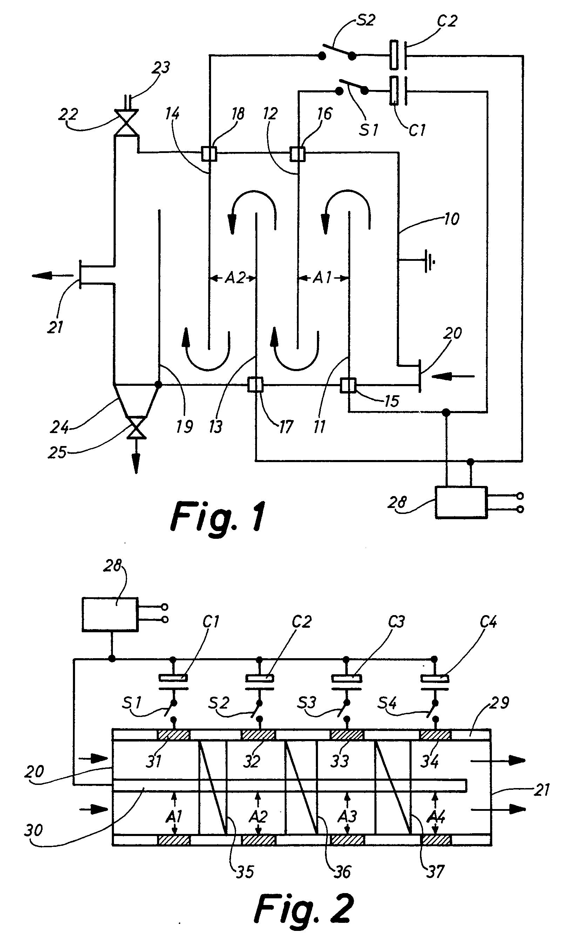

Die Vorrichtung der Fig. 1 ist besonders für die Behandlung flüssiger Stoffe bzw. Behandlung von in Flüssigkeit gelösten Stoffen geeignet. Ein Gehäuse 10 weist mehrere von der Gehäuseinnenwand nach innen hineinragende Wände 11, 12, 13, 14 auf, die als Elektroden, beispielsweise Stahl- oder Kohle-Elektroden ausgebildet sind. Weiterhin ragen diese Wände so in das Innere des Gehäuses 10 hinein, daß jeweils benachbarte Wände von gegenüberliegenden Seiten des Gehäuses abstehen. Die einzelnen Wände 11 ... 14 ragen dabei nur so weit in das Innere des Gehäuses 10 hinein, daß sie die gegenüberliegende Wand nicht erreichen. Durch diese Anordnung wird ein mäanderförmiger Zwangsweg für die zu behandelnden Stoffe geschaffen. Die Bewegungsrichtung ist hierbei durch Pfeile angegeben. Die Wände 11 ... 14 sind hierbei gegenüber dem elektrisch leitfähigen und geerdeten Gehäuse 10 elektrisch isoliert, was durch Isolatoren 15, 16, 17 und 18 erfolgt. Fig. 1 läßt noch eine weitere Wand 19 erkennen, die elektrisch mit dem Gehäuse 10 verbunden und damit geerdet ist. Diese Wand bildet noch eine Prall- bzw. Umlenkplatte zur Festlegung des Strömungsweges. Das Gehäuse 10 besitzt einen Einlaß 20, durch welchen die zu behandelnden Stoffe kontinuierlich oder diskontinuierlich zugeführt werden können. Von dort strömen sie den beschriebenen Strömungsweg entlang, bis sie nach Durchlaufen des Strömungsweges auf der stromabwärtigen Seite der Wand 19 zu einem Auslaß 21 gelangen. Dieser Auslaß 21 liegt in etwa auf halber Höhe des Gehäuses. Bei der Prinzipskizze der Fig. 1 handelt es sich im übrigen um eine Seitenansicht, d. h. die Flüssigkeit strömt zwischen den jeweiligen Platten senkrecht nach oben bzw. senkrecht nach unten. An der Oberseite des Gehäuses 10 nahe dem Auslaß 21 ist ein Entlüftungsventil 22 mit einem Luftauslaß 23 zur Atmosphäre vorgesehen. Gegenüberliegend hierzu ist auf der Unterseite des Gehäuses 10 im Bereich des Auslasses 21 ein Schlammauffänger 24 mit Auslaßventil 25 vorgesehen.

Im Ausführungsbeispiel der Fig. 1 bilden die Elektroden 11 und 12 sowie 13 und 14 jeweils ein Elektrodenpaar und damit zwischen sich eine Entladestrecke A1 bzw. A2. Bei diesem Ausführungsbeispiel sind die Elektroden 11 und 12 elektrisch mit einem ersten Kondensator C1 und die Elektroden 13 und 14 elektrisch mit einem zweiten Kondensator C2 verbindbar. Hierzu sind die Elektroden 11 und 13 jeweils unmittelbar mit einem Anschluß des zugeordneten Kondensators C1 bzw. C2 verbunden, während die Elektroden 12 und 14 über elektrische Schalter S1 bzw. S2 mit dem anderen Anschluß des zugeordneten Kondensators C1 bzw. C2 verbunden sind. Über eine Auflade- und Steuereinrichtung 28 können die Kondensatoren C1 und C2 auf vorbestimmte Spannungswerte aufgeladen werden. Werden die Schalter S1 oder S2 geschlossen, so entladen sich die Kondensatoren C1 bzw. C2 über die Entladestrecken A1 bzw. A2. Hierdurch wird dann ein Elektroimpuls hoher Energiedichte erzeugt. Die Kondensatoren C1 und C2 weisen nach der Erfindung unterschiedliche Kapazitätswerte auf, so daß sie bei ihrer Entladung Elektroimpulse unterschiedlicher Energiedichte erzeugen. Da die zu behandelnden Stoffe aufeinanderfolgend zuerst die Entladestrecke A1 und danach die Entladestrecke A2 passieren, werden sie in diesem Verlauf mit Elektroimpulsen unterschiedlicher Energiedichte beaufschlagt, wodurch die mit der Erfindung angestrebte höhere Ausbeute erreicht wird, Weiterhin kann durch die Steuereinheit 28 vorgesehen werden, daß die beiden Kondensatoren C1 und C2 auf unterscheidliche Spannungsamplituden aufgeladen werden. Auch ist es möglich, die Abstände der Elektroden der einzelnen Elektrodenpaare unterschiedlich groß zu gestalten, wodurch sich der Kapazitätswert der Entladestrecken verändert. Elektrisch gesehen bilden die Entladestrecken ja ebenfalls Kondensatoren bestehend aus einem Elektrodenpaar und dem zwischen ihnen vorbeifließenden elektrolytischen Stoff, der als Dielektrikum wirkt.

In Abhängigkeit von der Strömungsgeschwindigkeit der zu behandelnen Stoffe und in Abhängigkeit von der Schalthäufigkeit der Schalter S1 bzw. S2 kann dafür gesorgt werden, daß jedes Stoffpartikel mehrfach Gruppen von Elektroimpulsen der verschiedenen Energiedichten ausgesetzt wird. Schaltfrequenzen bis zu 50 Hz für die Schalter S1 bzw. S2 sind möglich. Impulszeiten von länger als 20 ms haben sich dagegen als ungünstig erwiesen, da dann Impulse erzeugt werden, deren Scheitelwert für längere Zeit gehalten wird.

Hierbei können dann jedoch bleibende Ionisierungen vorkommen, die unerwünscht sind.

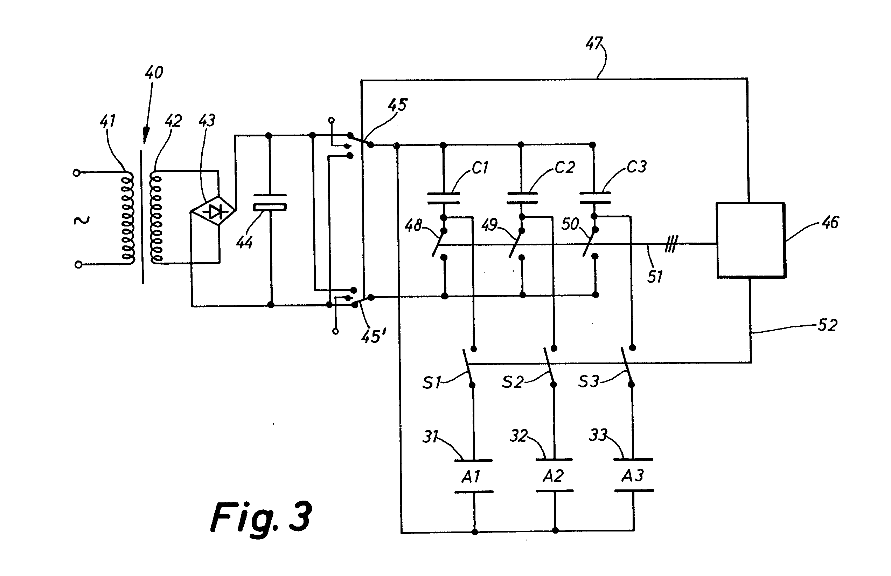

Als günstige Werte für die Kondensatoren C1 bzw. C2 haben sich Werte in der Größenordnung von 2,5—20 µF erwiesen, wobei die Kondensatoren auf Spannungen zwischen 8 und 12 kV aufgeladen werden. Als elektrische Schalter S1 bzw. S2 verwendet man günstig Thyristoren oder Thyratrons. Wie weiter unten im Zusammenhang mit Fig. 3 noch erläutert wird, ist wichtig, daß Aufladung und Entladung der Kondensatoren C1 bzw. C2 zeitlich getrennt voneinander erfolgen, da anderenfalls die Ladespannung direkt auf die Entladestrecke kommen könnte, was die Impulsdauer und -form der Elektroimpulse verfälschen würde. Daher ist Sorge dafür zu tragen, daß während der Auflading der Kondensatoren C1, C2 die Schalter S1 bzw. S2 geschlossen sind und umgekehrt, daß während der Entladung der Kondensatoren C1 und C2 (geschlossene Schalter S1 und S2) die Ladespannung aus der Steuervorrichtung 28 nicht zu den Kondensatoren gelangt.

Aus der Schaltung der Kondensatoren C1 bzw. C2 in Fig. 1 ist erkennbar, daß dort nur zwei Entladestrecken A1 und A2 vorgesehen sind, da die Kondensatoren C1 und C2 potentialmäßig voneinander getrennt sind. Legt man je einen Anschluß der beiden Kondensatoren auf gemeinsames Potential, beispielsweise durch elektrische Verbindung der Elektroden 11 und 13, so bilden die Elektroden 12 und 14 ein weiteres Elektrodenpaar, über welches sich der Kondensator C1 entladen kann.

Die Schalter S1 und S2 können synchron zueinander betätigt werde; es ist aber auch möglich, sie alternierend zu betätigen oder vollständig asynchron zueinander.

Durch die Kondensatorentladung werden in den zu behandelnden Stoffen elektromagnetische Felder hoher Energiedichte erzeugt. Dadurch, daß die Kondensatoren C1 und C2 unterschiedliche Werte haben, sind auch die Energiedichten der einzelnen Entladestrecken verschieden. Dies kann man auch dadurch erreichen, daß die Kondensatoren C1 bzw. C2 auf unterschiedliche Spannungsamplituden aufgeladen werden. Für manche Anwendungszwecke ist es nützlich, wenn die zu behandelnden Stoffe zunächst mit Impulsen niedriger Energiedichte und später mit Impulsen hoher Energiedichte beaufschlagt werden. Für solche Fälle ist dann der Kapazitätswert des Kondensators C1 kleiner (kleinere Energiedichte) als der des Kondensators C2 (größere Energiedichte). Im umgekehrten Falle, der bei manchen Anwendungszwecken ebenfalls sinnvoll ist, erhält man dann während des Durchlaufs der Stoffe fallende Energiedichten.

Fig. 2 zeigt schematisch eine Seitenansicht eines anderen Ausführungsbeispiels, das besonders zur Behandlung pastöser Stoffe geeignet ist. Hierzu ist ein Gehäuse 29 in Form eines zylindrischen Rohres oder eines mehreckigen langgestreckten Hohlkörpers 29 vorgesehen, der einen Einlaß 20 und einen Auslaß 21 besitzt. In der Mitte des Gehäuses 29 ist eine Stabförmige Elektrode 30 angeordnet, die in beliebiger Weise abgestützt ist. In den Gehäusewandungen sind, der Gehäuseform angepaßt, umlaufende Elektroden 31 bis 34 angeordnet, die beispielsweise im Falle eines zylindrischen Gehäuses 29 Ringelektroden sind. Zwischen diesen Elektroden und der gemeinsamen mittelelektrode 30 werden hier vier Entladestrecken gebildet. Als Impulsenergiequellen sind hier wiederum Kondensatoren C1 bis C4 vorgesehen, die über Schalter S1 bis S4 jeweils mit einer zugeordneten Elektrode 31 bis 34 verbindbar sind. Die Mittelelektrode 30 ist mit einem gemeinsamen Anschluß aller Kondensatoren C1 bis C4 verbinden. Eine Steuereinheit 28 sorgt wiederum für die Aufladung der Kondensatoren. Auch hier haben die Kondensatoren C1 bis C4 unterschiedliche Kapazitätswerte. Die pastösen Stoffe werden in Pfeilrichtung vom Einlaß 20 zum Auslaß 21 durch das Gehäuse 29 hindurchgedrückt, so daß sie im Verlauf ihres Weges die einzelnen Entladestrecken der Elektroden 31 bis 34 durchlaufen, wo sie wiederum mit Impulsen unterschiedlicher Energiedichte beaufschlagt werden.

Da für die Behandlung der Materialien auch die Polarität der Elektroimpulse bezogen auf die räumliche Ausrichtung der Stoffteilchen eine Rolle spielt, hat sich gezeigt, daß eine höhere Ausbeute dann erreicht werden kann, wenn die relative Ausrichtung zwischen den elektrischen Feldern und den Teilchen verändert wird. Hierzu ist nach einer Variante der Erfindung vorgesehen, daß die räumliche Ausrichtung der Teilchen während des Durchlaufs durch die Vorrichtung geändert wird. Dies kann durch Leitbleche 35, 36, 37 erreicht werden, die im "feldfreien" Raum zwischen benachbarten Elektroden angeordnet sind und die dafür sorgen, daß die Teilchen umgeschaufelt werden. Statt der starren Leitbleche 35, 36, 37 kann auf ein Schneckenförderer vorgesehen sein, der sich ganz oder abschnittsweise durch das Gehäuse 29 hindurcherstreckt.

Nach einer anderen Variante der Erfindung kann die relative Ausrichtung zwischen dem Feld und den Teilchen auch dadurch geändert werden, daß die einzelnen Kondensatoren jeweils für sich umgepolt werden oder daß aufeinanderfolgende Kondensatoren jeweils auf unterschiedliche Polarität aufgeladen werden.

Auch bei dem Ausführungsbeispiel der Fig. 2 ist es möglich, den Elektrodenabstand zu verändern. Man erhält dann einen sich verjüngenden oder sich erweiternden Durchlaufweg, wobei insbesondere im ersten Fall in Kombination mit dem erwähnten Schneckenförderer eine Schneckenpresse geschaffen werden kann. In diesem Falle kann man in den Gehäusewandungen auch kleinere Löcher vorsehen, durch welche ausgepreßte und/oder durch die Elektroimpulse freigesetzte Flüssigkeit abfließen kann.

Bei dem Ausführungsbeispiel der Fig. 2 können die Schalter S1 bis S4 synchron, asynchron, gleichphasig oder gegenphasig geschaltet werden.

Obwohl beide Ausfüngsbeispiele der Fig. 1 und 2 mehrere in Bewegungsrichtung der zu behandelnden Stoffe hintereinandergeschaltet Entladestrecken A1 bsw. A2 zeigen, kann die Erfindung auch mit einer einzigen Entladestrecke ausgerführt werden. Hierzu benötigt man dann nur ein Elektrodenpaar, das über zwei oder mehr Schalter mit der gleichen Anzahl von Kondensatoren verbindbar ist, die jeweils unterschiedliche Kapazitätswerte haben. Durch abwechselnde Betätigung der einzelnen Schalter kann die nur eine Entladestrecke mit Elektroimpulsen unterschiedlicher Energiedichte beaufschlagt werden.

Fig. 3 zeigt ein Prinzipschaltbild einer Steuer- und Aufladeschaltung zur Verwendung bei der Vorrichtung nach den Fig. 1 und 2. Es sei angenommen, daß drei Kondensatoren C1, C2 und C3 mit drei Entladestrecken A1, A2 bzw. A3 vorgesehen sind. Entsprechend sind drei Schalter S1, S2 und S3 vorhanden. Netzspannung wird über einen Transformator 40 mit der Primärwicklung 41 und der Sekundärwicklung 42 auf die gewünschte Ladespannung von beispielsweise 8 kV hochtransformiert und dann in einem Brückengleichrichter 43 gleichgerichtet. Die hochgespannte und gleichgerichtete Spannung wird zunächst in einem Glättungs- und Pufferkondensator 44 geglättet und zwischengespeichert. Über Wechselschalter 45 bzw. 45' wird die gleichgerichtete Hochspannung den Kondensatoren C1, C2 bzw. C3 zugeführt. Mit den Wechselschaltern 45 bzw. 45', die von einer Steuereinheit 46 über eine Steuerleitung 47 betätigbar sind, kann die Polarität der Ladespannung gewechselt werden, und es kann eine neutrale Zwischenstellung gewählt werden, in welcher die Kondensatoren C1 bis C3 vollständig von der Energiezufuhr aus dem Transformator 40 abgetrennt sind. In einer elektrischen Leitung von dem Schalter 45' zu jeweils einem Anschluß der Kondensatoren C1 bis C3 sind hier jeweils Schalter 48, 49 bzw. 50 vorgesehen, mit denen erreicht werden kann, daß nur der Kondensator, dessen Schalter geschlossen ist, aufgeladen wird, Hierdurch wird erreicht, daß nicht alle drei Kondensatoren C1 bis C3 gleichzeitig aufgeladen werden, so daß die Last für den Transformator 40 und damit das Netz begrenzt ist. Die Schalter 48 bis 50 können jeweils separat über Steuerleitungen 51 von der Steuereinheit 46 betätigt werden.

Für die Entladung der Kondensatoren sind die erwähnten Schalter S1 bis S3 vorgesehen, die über eine Steuerleitung 51 von der Steuereinheit 46 geöffnet bzw. geschlossen werden können.

Wie oben bereits beschrieben, muß dafür gesorgt werden, daß Aufladung und Entladung der Kondensatoren zeitlich getrennt erfolgen. Die Steuereinheit 46 ist daher so ausgebildet, daß die Schalter S1 bis S3 mindestens immer dann geöffnet sind, wenn die Schalter 45 und 45' in einer ihrer beiden Stellungen sind, in denen sie die gleichgerichtete Hochspannung den Kondensatoren zuführen. Ein Schließen der Schalter S1, S2 bzw. S3 ist dahen nur dann möglich, wenn die Schalter 45 und 45' in ihrer gezeigten Neutralstellung sind, in welcher die Kondensatoren von der Energiezufuhr abgesperrt sind.

In gleicher Weise sorgt die Steuereinheit 46 dafür, daß die Schalter 48, 49 bzw. 50 nur dann geschlossen sind, wenn auch die Schalter 45 und 45' in einer ihrer beiden Endlagen sind, während umgekehrt dafür gesorgt wird, daß die Schalter 48, 49 und 50 stets dann geöffnet sind, wenn zumindest einer der Schalter S1 bis S3 geschlossen ist.

Es ist ohne weiteres einsehbar, daß die Schalter 48 bis 50 auch fortgelassen werden können. In diesem Falle werden die drei Kondensatoren C1 bis C3 dann parallel aufgeladen.

Ein günstiger Nebeneffekt der Vorrichtung nach der Erfindung besteht darin, daß sie als kapazitiver Phasenschieber wirkt. Mit anderen Worten benötigt die Vorrichtung überwiegend kapazitiven Blindstrom. Dies ist insbesondere deswegen günstig, da der heute durch elektrische Motoren etc. betötigte Blindstrom aus dem Netz induktiv ist und durch aufwendige Maßnahmen beim Verbraucher oder aber im Elektrizitätswerk kompensiert werden muß.

Auch in dem Ausführungsbeispiel der Fig. 3 werden als Schalter elektronische Schalter in Form von Thyratrons oder Thyristoren verwendet.

Abschließend sei noch kurz auf die Form der erzeugten Elektroimpulse eingegangen. Diese Impulse haben sehr steile Anstiegsflanken (wenige μs), verweilen mit hochfrequenten Schwingungen kurzfristig (ebenfalls μs) auf dem Scheitelwert und fallen dann nach einer e-Funktion ab. Die Steilheit des Abfallens der e-Funktion hängt von der Endladezeitkonstante der Kondensatoren und der zugeordneten Entladestrecke ab. Die Entladung wird hierbei nicht völlstandig durchgeführt. Vielmehr öffnen die zugeordneten Schalter S1 bis S4 nach vorgegebener Zeit oder in Abhängigkeit von einem vorgegebenen Spannungswert, so daß in den Kondensatoren eine Restspannung bleibt. Die Energie der Elektroimpulse ist proportional dem Integral unter der Fläche der Impulse. Die Energiedichte (Energie pro Zeitenheit) hängt ab von der Breite der Impulse, welche wiederum von der Entladezeitkonstante bestimmte wird sowie dem Scheitelwert der Impulse.

Selbstverständlich ist es mit der Vorrichtung nach der Erfindung auch möglich, die Anzahl der Elektroimpulse mit den einzelnen Energiedichestufen beliebig zu variieren.

Beispielsweise können die Stoffe im Bereich der Elektrode 31 einer größeren Anzahl von Impulsen ausgesetzt werden als bei den übrigen Elektroden 32 bis 34 der Fig. 2.

Ergänzend zur Fig. 2 ist noch anzumerken, daß die Leitbleche 35 bis 37 auch fortgelassen werden können. In diesem Fall wird die Mittelelektrode 30 durch Abstandhalter gestützt, wobei diese als Isolatoren auszubilden sind. Auch ist es möglich, die Elektrodenflächen der Elektroden 31 bis 34 unterschiedlich groß auszulegen. Schließlich ist noch darauf hinzuweisen, daß nicht alle Kondensatoren jeweils gegenüber den anderen Kondensatoren unterschiedliche Werte haben müssen. So können beispielsweise die Kondensatoren C1 und C3 einerseits und die Kondensatoren C2 und C4 andererseits die gleichen Kapazitätswerte haben. Beim Durchlauf durch die Vorrichtung werden die Stoffe dann zweimal hintereinander jeweils der gleichen Energiesequenz ausgesetzt.

Statt eines Behandlungsraumes gemäß den Fig. 1 und 2 können auch zwei oder mehr separate Vorrichtungen der gezeigten Art hintereinandergeschaltet sein. Auch können einzelne Vorrichtungen der gezeigten Art in einer Behandlungsstrecke im "Bypass" geschaltet sein. Obwohl die Gehäuse und Verbindungsleitungen der einzelnen Vorrichtungen geerdet sind, kann der Elektrolyt selbst doch beträchtliche elektrische Feldstärken bzw. elektrische Energiedichten von einem Gehäuse zu den weiteren Gehäusen leiten. Um dies zu verhindern, werden im Bereich von Einlaß und/oder Auslaß der einzelnen Gehäuse zusätzliche Erdungen vorgesehen, beispielsweise in Form von geerdeten Sieben oder Lochplatten, so daß der dort hindurchströmende Elektrolyt im wesentlichen auf Erdpotential gezwungen wird.

Werden mehrere Vorrichtungen für eine Behandlungsstrecke verwendet, so kann jede Vorrichtung mit impulsen konstanter Energiedichte arbeiten, so daß die Impulsgruppen unterschiedlicher Energiedichte an räumlich getrennten Stellen auftreten. Natürlich ist es auch möglich, innerhalb jeder einzelnen Vorrichtung die Impulsgruppen unterschiedlicher Energiedichte zu erzeugen, wie oben beschrieben.

Sämtliche in den Ansprüchen, der Beschreibung und der Zeichnung dargestellten technischen Einzelheiten können sowohl für sich als auch in beliebiger Kombination miteinander erfindungswesentlich sein.

**Patentansprüche**

1. Elektroimpulsverfahren zur Behandlung von dispersen Systemen, zur Gewinnung einzelner Phasen aus den dispersen Systemen oder zur Abtötung von Bakterien,

bei dem die Stoffe in einem Elektrolyte mehrfach zeitlich aufeinanderfolgend Elektroimpulsen in Form von Kondensatorentladungen ausgesetzt werden,

dadurch gekennzeichnet,

daß man jede Volumeneinheit des behandelten dispersen Systems zeitlich nacheinander Gruppen von Elektroimpulsen aussetzt, die jeweils unterschiedliche Energiedichte haben.

2. Elektroimpulsverfahren nach Anspruch 1, dadurch gekennzeichnet, daß jede Gruppe von Elektroimpulsen zwischen 1 und n einzelne Elektroimpulse gleicher Energiedichte aufweist.

3. Elektroimpulsverfahren nach Anspruch 1 oder 2, dadurch gekennzeichnet, daß aufeinanderfolgende Impulsgruppen Elektroimpulse mit unterschiedlich langer Zeitdauer haben.

4. Elektroimpulsverfahren nach Anspruch 1, 2 oder 3, dadurch gekennzeichnet, daß aufeinanderfolgende Impulsgruppen Elektroimpulse mit unterschiedlicher Amplitude haben.

5. Elektroimpulsverfahren nach einem der Ansprüche 1 bis 4, dadurch gekennzeichnet, daß aufeinanderfolgende Impulsgruppen Elektroimpulse mit unterschiedlich steilen Anstiegsflanken haben.

6. Elektroimpulsverfahren nach einem der Ansprüche 1 bis 5, dadurch gekennzeichnet, daß aufeinanderfolgende Impulsgruppen Elektroimpulse mit steigender Energiedichte haben.

7. Elektroimpulsverfahren nach einem der Ansprüche 1 bis 5, dadurch gekennzeichnet, daß aufeinanderfolgende Impulsgruppen Elektroimpulse mit fallender Energiedichte haben.

8. Elektroimpulsverfahren nach einem der Ansprüche 1 bis 7, dadurch gekennzeichnet, daß der zeitliche Abstand aufeinanderfolgender Elektroimpulse größer als 20 ms ist.

9. Elektroimpulsverfahren nach einem der Ansprüche 1 bis 8, dadurch gekennzeichnet, daß die Energiedichte der Elektroimpulse so gewählt ist, daß keine bleibende Ionisierung (keine Radikale) auftritt.

10. Eletroimpulsverfahren nach Anspruch 9, dadurch gekennzeichnet, daß die Spannungsamplitude der Elektroimpulse zwischen 6 kV und 12 kV liegt.

11. Elektroimpulsverfahren nach einem der Ansprüche 1 bis 10, dadurch gekennzeichnet, daß die Elektroimpulse durch Entladung von Kondensatoren unterschiedlicher Kapazitätswerte erzeugt werden.

12. Elektroimpulseverfahren nach Anspruch 11, dadurch gekennzeichnet, daß die Elektroimpulse durch Entladung von auf unterschiedliche Spannung aufgeladenen Kondensatoren erzeugt werden.

13. Vorrichtung zur Durchführung des Verfahrens nach Anspruch 1, mit mindestens einer Behandlungskammer (10) mit einem Einlaß (20) und einem Auslaß (21), durch welche hindurch die Stoffe bewegt werden, mit mindestens einem in der Behandlungskammer (10) angeordneten Elektrodenpaar (11, 12; 13, 14; 31—34), wobei jedes Elektrodenpaar eine Entladestrecke (A1—A4) bildet, mit mindestens einem jedem Elektrodenpaar zugeordneten Kondensator (C1—C4), mit steuerbaren Schaltern (S1—S4) zum Verbinden der Kondensatoren (C1—C4) mit den Elektroden, mit Einrichtungen (28; 40—45) zum Aufladen der Kondensatoren, und mit einer Steuereinrichtung (46) zum Steuern der Schalter (S1—S4) dadurch gekennzeichnet, daß die Kondensatoren (C1—C4) derart unterschiedlich aufladbar sind, daß beim Schließen der Schalter (S1—S4) Elektroimpulse unterschiedlicher Energiedichte erzeugbar sind, und daß die Steuereinrichtung (46) derart ausgebildet ist, daß Gruppen von Elektroimpulsen jeweils unterschiedlicher Energiedichte erzeugbar sind.

14. Vorrichtung nach Anspruch 13, dadurch gekennzeichnet, daß die Kondensatoren (C1—C4) unterschiedliche Kapazitätswerte haben.

15. Vorrichtung nach einem der Ansprüche 13 oder 14, dadurch gekennzeichnet, daß die Einrichtungen (28; 40—45) zum Aufladen der Kondensatoren (C1—C4) derart ausgebildet sind, daß die Kondensatoren (C1—C4) auf unterschiedliche Spannungswerte aufladbar sind.

16. Vorrichtung nach einem der Ansprüche 13 bis 15, gekennzeichnet durch Einrichtungen (45, 46) zur Umsteuerung der Polarität der einzelnen Kondensatoren (C1—C4).

17. Vorrichtung nach einem der Ansprüche 13 bis 16, dadurch gekennzeichnet, daß die Elektroden (30—34; 11—14) der einzelnen Entladestrecken unterschiedliche Flächen und/oder Abstände aufweisen.

18. Vorrichtung nach Anspruch 17, dadurch gekennzeichnet, daß zwischen in Bewegungsrichtung der Stoffe hintereinander angeordneten Entladestrecken Vorrichtungen (35—37) vorhanden sind zur Umwälzung der Stoffe.

19. Vorrichtung nach Anspruch 18, dadurch gekennzeichnet, daß die Vorrichtungen zur Umwälzung der Stoffe Leitbleche (35—37) sind.

20. Vorrichtung nach Anspruch 19, dadurch gekennzeichnet, daß die Vorrichtungen zur Umwälzung der Stoffe einen Schneckenförderer enthalten.

21. Vorrichtung nach einem der Ansprüche 17 bis 20, dadurch gekennzeichnet, daß der Schneckenförderer zusammen mit den Elektroden eine Schneckenpresse bildet.

22. Vorrichtung nach einem der Ansprüche 13 bis 21, dadurch gekennzeichnet, daß zwei oder mehr Behandlungskammern in Bewegungsrichtung der Stoffe hintereinandergeschaltet sind, wobei jede Behandlungskammer mindestens ein Elektrodenpaar aufweist.

23. Vorrichtung nach Anspruch 22, dadurch gekennzeichnet, daß in der Strömungsverbindung der hintereinandergeschalteten Behandlungskammern eine Erdung vorgesehen ist, vorzugsweise in Form eines elektrisch geerdeten Siebes.

## Revendications

1. Procédé de traitement par impulsions électriques de systèmes dispersés, pour l'obtention de phases individuelles à partir de systèmes dispersés ou pour la destruction de bactéries, dans lequel les substances sont exposées, dans un électrolyte, à des impulsions électriques successives, répétées dans le temps, sous la forme de décharges de condensateur, caractérisé en ce que, successivement dans le temps, on expose chaque unité de volume du système dispersé traité à des groupes d'impulsions électriques qui ont chacun des densités de flux d'énergie différentes.

2. Procédé de traitement par impulsions électriques suivant la revendication 1, caractérisé en ce que chaque groupe d'impulsions électriques présente entre une et n impulsions électriques individuelles de même densité de flux d'énergie.

3. Procédé de traitement par impulsions électriques suivant l'une ou l'autre des revendications 1 et 2, caractérisé en ce que des groupes d'impulsions successifs ont des impulsions électriques qui présentent une durée de longueur différente.

4. Procédé de traitement par impulsions électriques suivant l'une ou l'autre des revendications 1, 2 et 3, caractérisé en ce que des groupes d'impulsions successifs ont des impulsions électriques qui présentent une amplitude différente.

5. Procédé de traitement par impulsions électriques suivant l'une quelconque des revendications 1 à 4, caractérisé en ce que des groupes d'impulsions successifs ont des impulsions électriques qui présentent des flancs ascendants de raideur différente.

6. Procédé de traitement par impulsions électriques suivant l'une quelconque des revendications 1 à 5, caractérisé en ce que des groupes d'impulsions successifs ont des impulsions électriques qui présentent une densité croissante de flux d'énergie.

7. Procédé de traitement par impulsions électriques suivant l'une quelconque des revendications 1 à 6, caractérisé en ce que des groupes d'impulsions successifs ont des impulsions électriques qui présentent une densité décroissante de flux d'énergie.

8. Procédé de traitement par impulsions électriques suivant l'une quelconque des revendications 1 à 7, caractérisé en ce que l'écart dans le temps entre des impulsions électriques successives est supérieur à 20 ms.

9. Procédé de traitement par impulsions électriques suivant l'une quelconque des revendications 1 à 8, caractérisé en ce que la densité de flux d'énergie des impulsions électriques est choisie de façon qu'aucune ionisation permanente (aucun radical) n'apparaisse.

10. Procédé de traitement par impulsions électriques suivant la revendication 9, caractérisé en ce que l'amplitude de la tension des impulsions électriques se situe entre 6 kV et 12 kV.

11. Procédé de traitement par impulsions électriques suivant l'une quelconque des revendications 1 à 10, caractérisé en ce que les impulsions électriques sont produites par une décharge de condensateurs à valeur de capacité différente.

12. Procédé de traitement par impulsions électriques suivant la revendication 11, caractérisé en ce que les impulsions électriques sont produites par décharge de condensateurs chargés à des tensions différentes.

13. Dispositifs pour la mise en oeuvre du procédé suivant le revendication 1, comprenant au moins une chambre de traitement (10) qui possède une entrée (20) et une sortie (21) et à travers laquelle sont déplacées les substances, au moins une paire d'électrodes (11, 12; 13, 14; 31 à 34) agencée dans la chambre de traitement (10), chaque paire d'électrodes formant une section de décharge (A1 à A4), au moins un condensateur (C1 à C4) associé à chaque paire d'électrodes, des commutateurs (S1 à S4) qui peuvent être commandés, en vue de la connexion des condensateurs (C1 à C4) avec les électrodes, des dispositifs (28; 40 à 45) pour charger les condensateurs et un dispositif de commande (46) pour commande les commutateurs (S1 à S4), caractérisé en ce que les condensateurs (C1 à C4) peuvent être chargés différemment de façon qu'à la fermeture des commutateurs (S1 à S4) des impulsions électriques de différentes densités de flux d'énergie puissent être produites et en ce que le dispositif de commande (46) est réalisé de façon que des groupes d'impulsions électriques de densités de flux d'énergie respectivement différentes puissent être produits.

14. Dispositif suivant la revendication 13, caractérisé en ce que les condensateurs (C1 à C4) ont des valeurs de capacité différentes.

15. Dispositif suivant l'une ou l'autre des revendications 13 et 14, caractérisé en ce que les dispositifs (28; 40 à 45) destinés à la charge des condensateurs (C1 à C4) sont réalisés de façon que les condensateurs (C1 à C4) puissent être chargés à des valeurs de tension différentes.

16. Dispositif suivant l'une quelconque des revendications 13 à 15, caractérisé par les dispositifs (45, 46) d'inversion de la polarité des condensateurs individuels (C1 à C4).

17. Dispositif suivant l'une quelconque des revendications 13 à 16, caractérisé en ce que les électrodes (30 à 34; 11 à 14) des sections de décharge individuelles présentent des surfaces et/ou des écartements différents.

18. Dispositif suivant la revendication 17, caractérisé en ce que des dispositifs (35 à 37) de mise en circulation des substances sont présents entre des sections de décharge agencées l'une derrière l'autre dans le sens de déplacement des substances.

19. Dispositif suivant la revendication 18, caractérisé en ce que les dispositifs de mise en circulation des substances sont des plaques de guidage.

20. Dispositif suivant la revendication 19, caractérisé en ce que les dispositifs de mise en circulation des substances comportent un convoyeur à vis.

21. Dispositif suivant l'une quelconque des revendications 17 à 20, caractérisé en ce que le convoyeur à vis forme avec les électrode une extrudeuse à vis.

22. Dispositif suivant l'une ou l'autre des revendications 13 à 21, caractérisé en ce que deux ou plusieurs chambres de traitement sont montées en série dans le sens du déplacement des substances, chaque chambre de traitement présentant au moins une paire d'électrodes.

23. Dispositif suivant la revendication 22, caractérisé en ce que, dans le raccordement permettant un écoulement entre les chambres de traitement montées en série, une prise de terre est prévue, de préférence sous la forme d'un tamis mis électriquement à la terre.

**Claims**

1. Electric-impulse method for treating dispersed systems for obtaining individual phases from the dispersed systems or for destroying bacteria, in which method the substances are exposed in an electrolyte several times successively in time to electric impulses in the form of capacitor discharges, characterized by the fact that each volume unit of the dispersed system treated is exposed successively in time to groups of electric pulses which in each case have a different energy density.

2. Electric-impulse method according to Claim 1, characterized in that each group of electric impulses has between 1 and n individual electric impulses of the same energy density.

3. Electric-impulse method according to Claim 1 or 2, characterized in that successive impulse groups have electric impulses of varying duration in time.

4. Electric-impulse method according to Claim 1, 2 or 3, characterized in that successive impulse groups have electric impulses of varying amplitude.

5. Electric-impulse method according to Claims 1 to 4, characterized in that successive impulse groups have electric impulses having rising edges of varying steepness.

6. Electric-impulse method according to one of Claims 1 to 5, characterized in that successive impulse groups have electric impulses of increasing energy density.

7. Electric-impulse method according to one of Claims 1 to 5, characterized in that successive impulse groups have electric impulses of decreasing energy density.

8. Electric-impulse method according to one of Claims 1 to 7, characterized in that the time interval between successive electric impulses is greater than 20 ms.

9. Electric-impulse method according to one of Claims 1 to 8, characterized in that the energy density of the electric impulses is selected in such a manner that no residual ionization (no radical) occurs.

10. Electric-impulse method according to Claim 9, characterized in that the voltage amplitude of the electric impulses is between 6 kV and 12 kV.

11. Electric-impulse method according to one of Claims 1 to 10, characterized in that the electric impulses are generated by discharging capacitors having varying values of capacitance.

12. Electric-impulse method according to Claim 11, characterized in that the electric impulses are generated by discharging capacitors which have been charged up to varying voltages.

13. Device for carrying out the method according to Claim 1, comprising at least one treatment chamber (10) having an inlet (20) and an outlet (21) through which the substances are moved, at least one pair of electrodes (11, 12; 13, 14; 31—34) arranged in the treatment chamber (10), each pair of electrodes forming a discharge path (A1—A4), at least one capacitor (C1—C4) which is associated with each pair of electrodes, controllable switches (S1—S4) for connecting the capacitors (C1—C4) to the electrodes, devices (28; 40—45) for charging the capacitors, and with a control device (46) for controlling the switches (S1—S4), characterized in that the capacitors (C1—C4) can be differently charged in such a manner that when the switches (S1—S4) are closed, electric impulses of different energy density can be generated, and that the control device (46) is constructed such that groups of electric impulses having in each case a different energy density can be generated.

14. Device according to Claim 13, characterized in that the capacitors (C1—C4) have varying values of capacitance.

15. Device according to either of Claims 13 or 14, characterized in that arrangements (28; 40—45) for charging the capacitors (C1—C4) are of such a type that the capacitors (C1—C4) can be charged to varying voltage values.

16. Device according to one of Claims 13 to 15, characterized by arrangements (45, 46) for reversing the polarity of the individual capacitors (C1—C4).

17. Device according to one of Claims 13 to 16, characterized in that the electrodes (30—34; 11—14) of the individual discharge paths have varying areas and/or spacings.

18. Device according to Claim 17, characterized in that between discharge paths, arranged behind each other in the direction of movement of the substances, there are devices (35—37) for circulating the substances.

19. Device according to Claim 18, characterized in that the devices for circulating the substances are guide plates (35—37).

20. Device according to Claim 19, characterized in that the devices for circulating the substances contain a screw conveyor.

21. Device according to Claim 17 to 20, characterized in that the screw conveyor, in conjunction with the electrodes, forms a screw press.

22. Device according to one of Claims 13 to 21, characterized in that two or more treatment chambers are connected behind each other in the direction of movement of the substances, each treatment chamber being provided with at least one pair of electrodes.

23. Device according to Claim 22, characterized in that earthing, preferably in the form of an electrically earthed screen, is provided in the flow connexion of the treatment chambers connected behind each other.

**Fig. 1**

**Fig. 2**

*Fig. 3*